(19) Europäisches Patentamt
European Patent Office
Office européen des brevets

(11) Veröffentlichungsnummer : **0 053 717**
**B1**

(12) # EUROPÄISCHE PATENTSCHRIFT

(45) Veröffentlichungstag der Patentschrift : **29.02.84**

(51) Int. Cl.³ : **C 07 D307/94**, A 61 K 7/46, C 11 B 9/00

(21) Anmeldenummer : **81109208.9**

(22) Anmeldetag : **29.10.81**

(54) **Terpenderivate, deren Herstellung, Verwendung als Riechstoffe sowie diese enthaltende Riechstoffkompositionen.**

(30) Priorität : **06.12.80 DE 3046068**

(43) Veröffentlichungstag der Anmeldung : **16.06.82 Patentblatt 82/24**

(45) Bekanntmachung des Hinweises auf die Patenterteilung : **29.02.84 Patentblatt 84/09**

(84) Benannte Vertragsstaaten : **CH FR GB LI NL**

(56) Entgegenhaltungen :
**DE-B- 2 504 618**
**DE-B- 2 527 102**

(73) Patentinhaber : **Henkel Kommanditgesellschaft auf Aktien**
**Postfach 1100 Henkelstrasse 67**
**D-4000 Düsseldorf-Holthausen (DE)**

(72) Erfinder : **Upadek, Horst, Dr.**
**Kempenweg 18A**
**D-4006 Erkrath 1 (DE)**
Erfinder : **Bruns, Klaus, Dr.**
**Notburgaweg 6**
**D-4150 Krefeld-Traar (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 053 717 B1

Jouve, 18, rue St-Denis, 75001 Paris, France

Terpenderivate, deren Herstellung, Verwendung als Riechstoffe sowie diese enthaltende Riechstoffkompositionen

Gegenstand der Erfindung sind neue Terpenderivate, deren Herstellung, Verwendung als Riechstoffe sowie diese enthaltende Riechstoffkompositionen.

Wegen der ungleichmäßigen Verfügbarkeit vieler natürlicher Riechstoffkomponenten und der notwendigen Anpassung an wechselnde modische Geschmacksrichtungen hat die Riechstoffindustrie ständig Bedarf an neuen Riechstoffen, die entweder allein oder in Form von Kompositionen wertvolle Parfums mit interessanten Duftnoten darstellen. Da aufgrund wenig bekannter Zusammenhänge zwischen Struktur und Riechstoffeigenschaften die gezielte Synthese von Riechstoffen mit gewünschter olfaktorischer Qualität nicht möglich ist, besteht die Aufgabe, solche Verbindungen aufzufinden, die wertvolle Riechstoffeigenschaften aufweisen.

Es wurde gefunden, daß das Gemisch der Isomeren 6(9)-Isopropyl-9(6)-methyl-6,9-ethano-2-oxaspiro [4,5] dec-7-ene der allgemeinen Formel I

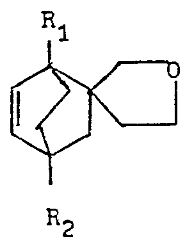

(I)

in welcher $R_1$ und $R_2$ entweder eine Methyl- oder eine Isopropyl-Gruppe bedeuten und $R_1$ ungleich $R_2$ ist, einen animalisch-holzigen, an trischen Tabak erinnernden Geruch besitzt und daher einen wertvollen neuen Riechstoff darstellt.

Diese Substanzen sind bisher nicht bekannt. Die Herstellung der erfindungsgemäßen Verbindungen in der Form eines Gemisches der Struktur- und Stereoisomeren erfolgt nach an sich bekannten Syntheseverfahren der organischen Chemie, die jedoch in der erfindungsgemäßen Reaktionsfolge und in Bezug auf die neuen Verbindungen ein neues Herstellungsverfahren darstellen. Weiterhin wurden solche Reaktionsbedingungen gefunden, die die Herstellung der erfindungsgemäßen Verbindungen in besonders guten Ausbeuten und in der für einen Riechstoff erforderlichen Reinheit ermöglichen.

Als Ausgangsmaterial für die Synthese dienen α-Terpinen II und Itaconsäureanhydrid III. Diese werden in einer Diels-Alder-Addition zum Addukt IV umgesetzt. Dabei entsteht ein Gemisch aus den beiden Strukturisomeren IVa und IVb. Die Diels-Alder-Reaktion kann in besonders vorteilhafter Weise ohne Lösungsmittel unter Verwendung eines Überschusses von 10-20 Mol.% an α-Terpinen, bezogen auf das Itaconsäureanhydrid, bei einer Temperatur von 140-195 °C durchgeführt werden. Durch Reduktion des Diels-Alder-Adduktes IV mit einem komplexen Metallhydrid gelangt man zu dem Diol V, welches wiederum aus den Strukturisomeren Va und Vb zusammengesetzt ist. Diese Reduktion verläuft bei Verwendung von Natriumborhydrid in Isopropanol nur unvollständig. Leichter gelingt die Reduktion, wenn als komplexes Metallhydrid das leicht handhabbare Natrium-bis-(2-methoxy-ethoxy)-aluminiumdihydrid verwendet wird, welches als 70 %ige Lösung in Toluol im Handel unter dem Warenzeichen Vitride [(R)] der Firma Hexcel Speciality Chemicals erhältlich ist. Schon mit einem geringen Überschuß von ca. 10 Mol.% dieses Reduktionsmittels gelingt eine praktisch vollständige Reduktion des Adduktes IV zum Diol V.

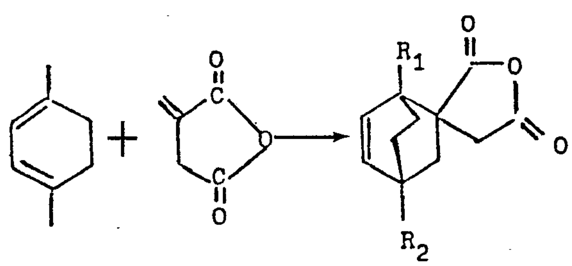

IVa : $R_1 = CH_3$
$R_2 = i\text{-}C_3H_7$
IVb : $R_1 = i\text{-}C_3H_7$
$R_2 = CH_3$

(II) (III) (IV)

(IV)

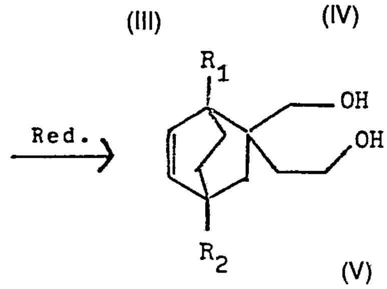


Va : $R_1 = CH_3$
$R_2 = i\text{-}C_3H_7$
Vb : $R_1 = i\text{-}C_3H_7$
$R_2 = CH_3$

(V)

(V) 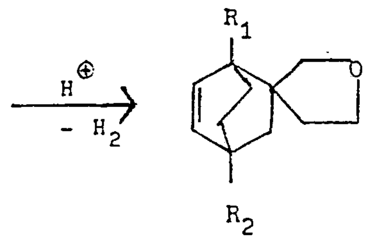


(I)

Ia : $R_1 = CH_3$
$R_2 = i\text{-}C_3H_7$

Ib : $R_1 = i\text{-}C_3H_7$
$R_1 = CH_3$

Die Diole V werden, ohne Isolation aus dem Reaktionsgemisch, durch Zusatz eines sauren Katalysators, z. B. von para-Toluolsulfonsäure, unter Wasserabspaltung zu den cyclischen Ethern der 6(9)-Isopropyl-9(6)-methyl-6,9-ethano-2-oxaspiro [4,5] dec-7-ene I umgesetzt. Neben den beiden strukturisomeren Verbindungen Ia und Ib entstehen bei diesem Syntheseverfahren auch die bezüglich des Spirozentrums 5 beiden jeweils möglichen Diastereomeren.

Das Isomerengemisch der 6(9)-Isopropyl-9(6)-methyl-6,9-ethano-2-oxaspiro [4,5] dec-7-ene zeichnet sich durch eine animalisch-holzige, an frischen Tabak erinnernde Geruchsnote sowie durch eine gute Haftfestigkeit aus. Ein weiterer Vorteil ist die sehr gute Kombinationsfähigkeit des neuen Riechstoffes zu neuartigen Riechstoffkompositionen, denen er eine interessante holzige Geruchsnuance verleiht.

Das Isomerengemisch der 6(9)-Isopropyl-9(6)-methyl-6,9-ethano-2-oxaspiro [4,5] dec-7-ene kann mit anderen Riechstoffen in verschiedensten Mengenverhältnissen zu neuen Riechstoffkompositionen gemischt werden. Im allgemeinen wird sich sein Anteil in den Riechstoffkompositionen in den Mengen von 1 bis 50 Gew.-%, bezogen auf die gesamte Komposition, bewegen. Derartige Kompositionen können zur Parfümierung von kosmetischen Präparaten, wie z. B. Duftwässern, Aerosolen, Badepräparaten, Shampoos, Seifen, Lotionen und Cremes, sowie in der Extrait-Parfümerie verwendet werden. Sie können aber auch zur Geruchsverbesserung technischer Produkte, wie z. B. Wasch- und Reinigungsmittel, Textilbehandlungsmittel und Wäscheweichspüler, eingesetzt werden. Zur Parfümierung der verschiedenen Produkte werden diesen die Kompositionen im allgemeinen in Konzentrationen von 0,05 bis 2 Gew.-%, bezogen auf das gesamte Produkt, zugesetzt.

Die nachfolgenden Beispiele sollen den Gegenstand der Erfindung näher erläutern, ohne ihn jedoch hierauf zu beschränken :

## Beispiele

### Beispiel 1

Herstellung von 6(9)-Isopropyl-9(6)-methyl-6,9-ethano-2-oxaspiro [4,5] dec-7-en in Form des Gemisches von Struktur- und Stereoisomeren.

a) Herstellung des Diels-Alder-Adduktes IV aus α-Terpinen und Itaconsäureanhydrid.

490,5 g (3,6 Mol) α-Terpinen und 336,3 g (3 Mol) Itaconsäureanhydrid wurden 4 Stunden auf 145-160 °C erhitzt. Nach Abdestillation des überschüssigen α-Terpinens wurden bei 145-156 °C/1,5 mbar 538 g Addukt IV gesammelt.

b) Reduktion des Diels-Alder-Adduktes IV und Cyclisierung zum 6(9)-Isopropyl-9(6)-methyl-6,9-ethano-2-oxaspiro [4,5] dec-7-en I.

Zu 24,8 g (0,1 Mol) des Adduktes IV in 67 ml Toluol wurden 61,6 ml « Vitride$^{(R)}$ » (0,22 Mol) in 45 Minuten unter Eiskühlung und Stickstoff-Atmosphäre bei 25-35 °C Reaktionstemperatur zugetropft. Es wurde 2 Stunden unter Rückfluß nachgerührt und die abgekühlte, klare Reaktionslösung unter guter Kühlung langsam so mit 420 ml 6 n-Salzsäure zersetzt, daß die Temperatur 50 °C nicht überschritt. Es wurde so lange nachgerührt, bis alle festen Bestandteile gelöst waren, die Toluol-Phase wurde abgetrennt und einmal mit Wasser nachgewaschen. Die organische Phase enthielt ca. 18 g Diol V und wurde direkt zur Dehydratisierung eingesetzt. Nach Zusatz von 1,8 g p-Toluolsulfonsäure zur Toluol-Lösung wurde unter Rückfluß das Wasser vollständig entfernt. Die abgekühlte Lösung wurde mit verdünnter Natronlauge neutralisiert, einmal mit Wasser gewaschen, eingeengt und destilliert.

Bei 73-82 °C/0,1 mbar wurden 11,9 g 6(9)-Isopropyl-9(6)-methyl-6,9-ethano-2-oxaspiro [4,5] dec-7-en I isoliert.

$n_D^{20} = 1{,}503\,4$

IR (Film) : u. a. 3 035 ( = CH), 1 372 und 1 390 (Isopropyl), 698 (cis-RCH = CHR') $cm^{-1}$

$^1H$-NMR ($CDCl_3$) : = 5,87-6,21 (m, 2H, olefinische H), 3,32-4,05 (m, 4H, —$CH_2$—O—), 0,75-2,30 (m, 18H)

Geruch : animalisch-holzig, an frischen Tabak erinnernd.

Beispiel 2

Blumig, holziger Komplex für Schaumbadformulierungen

| | |
|---|---|
| 6(9)-Isopropyl-9(6)-methyl-6,9-ethano-2-oxaspiro [4,5] dec-7-en | 110 Gew.-Teile |
| Cyclamber[R], (Henkel) | 70 Gew.-Teile |
| Phenylethylalkohol | 200 Gew.-Teile |
| Citronellol | 120 Gew.-Teile |
| Linalylacetat | 75 Gew.-Teile |
| α-Isomethyljonon | 70 Gew.-Teile |
| α-Hexylzimtaldehyd | 60 Gew.-Teile |
| Ylang-Ylangöl kstl. | 60 Gew.-Teile |
| Benzylacetat | 50 Gew.-Teile |
| Cyclopentadecanolid 10 % i. DEP | 40 Gew.-Teile |
| Methyldihydrojasmonat | 40 Gew.-Teile |
| Amylsalicylat | 30 Gew.-Teile |
| Vetivenol | 30 Gew.-Teile |
| Neroliöl, kstl. | 20 Gew.-Teile |
| Heliotropin | 15 Gew.-Teile |
| Blätteralkohol 10 % i. DEP | 10 Gew.-Teile |
| | 1 000 Gew.-Teile |

(DEP = Diethylphthalat)

## Ansprüche

1. 6(9)-Isopropyl-9(6)-methyl-6,9-ethano-2-oxaspiro [4,5] dec-7-ene der allgemeinen Formel I

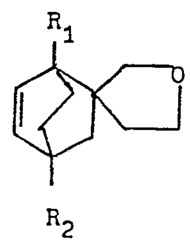

(I)

in welcher $R_1$ und $R_2$ entweder eine Methyl- oder eine Isopropyl-Gruppe bedeuten und $R_1$ ungleich $R_2$ ist.

2. Verfahren zur Herstellung von 6(9)-Isopropyl-9(6)-methyl-6,9-ethano-2-oxaspiro [4,5] dec-7-enen der allgemeinen Formel I mit der in Anspruch 1 angegebenen Bedeutung für $R_1$ und $R_2$ in Form eines Gemisches der Struktur- und Stereoisomeren, gekennzeichnet dadurch, daß man

a) Itaconsäureanhydrid und α-Terpinen nach Diels-Alder umsetzt,

b) das so erhaltene Diels-Alder-Addukt mit einem komplexen Metallhydrid zum 1(4)-Isopropyl-4-(1)-methyl-2-hydroxyethyl-2-hydroxymethyl-bicyclo [2.2.2] oct-5-en umsetzt und

c) dieses in Gegenwart eines sauren Katalysators zum 6(9)-Isopropyl-9(6)-methyl-6,9-ethano-2-oxaspiro [4,5] dec-7-en cyclisiert.

3. Verfahren nach Anspruch 2, dadurch gekennzeichnet, daß die Diels-Alder-Addition ohne Lösungsmittel und unter Einsatz eines Überschusses von 10-20 Mol.% an α-Terpinen bei einer Temperatur von 140-195 °C durchgeführt wird.

4. Verfahren nach Anspruch 2, dadurch gekennzeichnet, daß die Reduktion des Diels-Alder-Adduktes unter Verwendung von Natrium-bis-(2-methoxy-ethoxy)-aluminiumdihydrid durchgeführt wird.

5. Verwendung des Gemisches der isomeren 6(9)-Isopropyl-9(6)-methyl-6,9-ethano-2-oxaspiro [4,5] dec-7-ene der allgemeinen Formel I nach Anspruch 1 als Riechstoff.

6. Riechstoffkompositionen, dadurch gekennzeichnet, daß sie das Isomerengemisch der 6(9)-Isopropyl-9(6)-methyl-6,9-ethano-2-oxaspiro [4,5] dec-7-ene der allgemeinen Formel I nach Anspruch 1 in einer Menge von 1-50 Gewichtsprozenten, bezogen auf die gesamte Komposition, enthalten.

## Claims

1. 6(9)-isopropyl-9(6)-methyl-6,9-ethano-2-oxaspiro [4,5] dec-7-enes corresponding to the following general formula

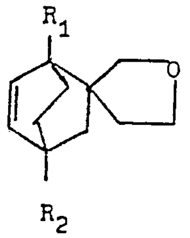

(I)

in which $R_1$ and $R_2$ represent a methyl group or an isopropyl group and $R_1$ is different from $R_2$.

2. A process for producing the 6(9)-isopropyl-9(6)-methyl-6,9-ethano-2-oxaspiro [4,5] dec-7-enes corresponding to general formula I with the meanings for $R_1$ and $R_2$ defined in Claim 1 in the form of a mixture of the structural and stereo isomers, characterized by the reaction sequence a-c :

a) Diels-Alder addition of itaconic acid anhydride and α-terpinene,

b) reduction of the Diels-Alder adduct with a complex metal hydride to 1(4)-isopropyl-4-(1)-methyl-2-hydroxyethyl-2-hydroxymethyl-bicyclo [2.2.2] oct-5-ene,

c) cyclization of the 1(4)-isopropyl-4(1)-methyl-2-hydroxyethyl-2-hydroxymethyl-bicyclo [2.2.2] oct-5-ene to form 6(9)-isopropyl-9(6)-methyl-6,9-ethano-2-oxaspiro [4,5] dec-7-ene in the presence of an acidic catalyst.

3. A process as claimed in Claim 2, characterized in that the Diels-Alder addition is carried out in the absence of a solvent at a temperature in the range from 140 to 195 °C using an excess of from 10 to 20 mole percent of α-terpinene.

4. A process as claimed in Claim 2, characterized in that reduction of the Diels Alder adduct is carried out using sodium-bis-(2-methoxyethoxy)-aluminium dihydride.

5. The use of the isomer mixture of 6(9)-isopropyl-9(6)-methyl-6,9-ethano-2-oxaspiro [4,5] dec-7-enes corresponding to general formula I in Claim 1 as a perfume.

6. Perfume compositions, characterized in that they contain the isomer mixture of the 6(9)-isopropyl-9(6)-methyl-6,9-ethano-2-oxaspiro [4,5] dec-7-enes corresponding to general formula I in Claim 1 in a quantity of from 1 to 50 % by weight, based on the composition as a whole.

**Revendications**

1. 6(9)-isopropyl-9(6)-méthyl-6,9-éthano-2-oxaspiro [4,5] déca-7-ènes de formule générale I

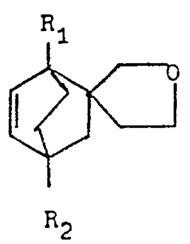

(I)

dans laquelle $R_1$ et $R_2$ représentent un groupe méthyle ou isopropyle et $R_1$ et $R_2$ sont différents.

2. Procédé de préparation des 6(9)-isopropyl-9(6)-méthyl-6,9-éthano-2-oxaspiro [4,5] déca-7-ènes de formule générale I dans laquelle $R_1$ et $R_2$ ont la signification indiquée dans la revendication 1 à l'état de mélange d'isomères de structure et de stéréoisomères, caractérisé par la séquence de réactions a) à c) :

a) addition selon Diels-Alder de l'anhydride itaconique et de l'alpha-terpinène,

b) réduction de l'adduct de Diels-Alder à l'aide d'un hydrure métallique complexe en le 1(4)-isopropyl-4-(1)-méthyl-2-hydroxyéthyl-2-hydroxyméthyl-bicyclo [2.2.2] octa-5-ène,

c) cyclisation du 1(4)-isopropyl-4-(1)-méthyl-2-hydroxyéthyl-2-hydroxyméthyl-bicyclo [2.2.2] octa-5-ène en le 6(9)-isopropyl-9(6)-méthyl-6,9-éthano-2-oxaspiro [4,5] déca-7-ène en présence d'un catalyseur acide.

3. Procédé selon la revendication 2, caractérisé en ce que l'addition de Diels-Alder est effectuée sans solvant et avec utilisation d'un excès de 10 à 20 moles% de l'alpha-terpinène à une température de 140 à 195 °C.

4. Procédé selon la revendication 2, caractérisé en ce que la réduction de l'adduct de Diels-Alder est effectuée à l'aide du dihydrure de sodium et de bis-(2-méthoxyéthoxy)-aluminium.

5. Utilisation du mélange des isomères 6(9)-isopropyl-9(6)-méthyl-6,9-éthano-2-oxaspiro [4,5] déca-7-ènes de formule générale I selon la revendication 1 en tant que matière aromatique.

6. Compositions de parfums caractérisées en ce qu'elles contiennent le mélange des isomères 6(9)-isopropyl-9(6)-méthyl-6,9-éthano-2-oxaspiro [4,5] déca-7-ènes de formule générale I selon la revendication 1 en quantité de 1 à 50 % en poids, par rapport à la composition totale.